# EUROPEAN PATENT APPLICATION

(11) **EP 4 183 390 A1**
(43) Date of publication of application: **24.05.2023**
(21) Application number: 22152174.3
(22) Date of filing: 19.01.2022
(51) Int. Cl.: A61K 9/20, A61K 31/444, A61P 7/02

(54) **AN ORODISPERSIBLE PHARMACEUTICAL DOSAGE FORM OF EDOXABAN**

(30) Priority: 22.11.2021 IN 202121053646
(71) Applicant: Intas Pharmaceuticals Limited, Ahmedabad - 380054, Gujrat (IN)
(72) Inventor: Pérez Pérez, Jesús Manuel, Sevilla (ES); Márquez Quidiello, María Teresa, Sevilla (ES); Muñoz Ruiz, Angel José, Sevilla (ES); Lluch Lores, María Carmen, Barcelona (ES); Formosa Márquez, Xavier, Barcelona (ES)
(74) Representative: Ter Meer Steinmeister & Partner

(57) **Abstract**

The present invention relates to an orodispersible pharmaceutical dosage form of edoxaban having overall improved characteristics, its process of manufacturing and its use as anticoagulant.

## Description

The present invention relates to an orodispersible pharmaceutical dosage form of edoxaban having overall improved characteristics, its process of manufacturing and its use as anticoagulant.

### Background Art

Edoxaban is represented by the structural formula (I) and its chemical name is (N'-(5-chloropyridin-2-yl)-N-[(1S,2R,4S)-4-(dimethylcarbamoyl)-2-[(5-methyl-6,7-dihydro-4H-[1,3]thiazolo[5,4-c]pyridine-2-carbonyl)amino]cyclohexyl]oxamide).

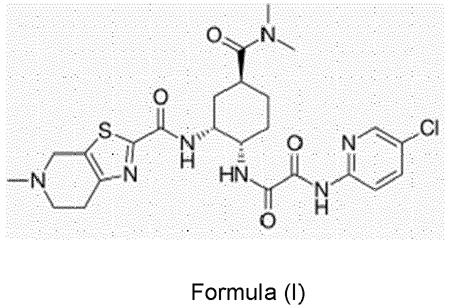

In Europe, edoxaban (Lixiana^{®}) was approved by the European Medicines Agency (EMA) in June 2015, whereas in the US, the Food and Drug Administration (FDA) approved edoxaban (Savaysa^{®}) in January 2015. Edoxaban is indicated both in EU and the US in: (i) prevention of stroke and systemic embolism in adult patients with nonvalvular atrial fibrillation (NVAF) with one or more risk factors, such as congestive heart failure, hypertension, age ≥ 75 years, diabetes mellitus, prior stroke or transient ischaemic attack (TIA), and (ii) treatment of deep vein thrombosis (DVT) and pulmonary embolism (PE), and for the prevention of recurrent DVT and PE in adults. Lixiana^{®}/Savaysa^{®} is commercially available both in Europe and the US as immediate release film-coated tablets in 15, 30 and 60 mg strengths (based on edoxaban free base). The used pharmaceutically acceptable salt of edoxaban in Lixiana^{®}/Savaysa^{®} is edoxaban tosylate monohydrate.

Edoxaban immediate-release film-coated tablets are the only available commercial dosage form in Europe (Lixiana^{®}) and in the US (Savaysa^{®}). These conventional tablets need to be swallowed - meaning that patients in therapy should be able to perform the action of swallowing correctly. However, swallowing may prove difficult for some category of patients, for example, for elderly patients or pediatric patients, or for patients who barely cooperate with medical personnel because of progression of disabling diseases. In these clinical situations the patient has difficulty in swallowing, and therefore it would be advisable to replace the tablets that are to be swallowed with water with other oral dosages forms in which swallowing is made easier.

A particular solid pharmaceutical dosage form that rapidly disintegrates are orodispersible tablets (also named orally disintegrated tablet (ODT) or fast disintegrating tablets), which do not require water, and hence can be consumed in situations where patients require an easy-to-administer dosage form. Orodispersible tablets are an improved dosage form with respect to conventional tablets, as they are not required to be swallowed and are in line with the current fast-paced modern lifestyle. Furthermore, orodispersible tablets are especially suitable for patients suffering from dysphagia, wherein closure of the glottis and simultaneous contraction of the muscles of the larynx do not propel adequately the conventional tablet that needs to be swallowed.

In this regard, there are disclosures in the prior art directed to orodispersible pharmaceutical compositions comprising edoxaban. However, these suffer from the serious drawbacks of being manufactured by wet-granulation processes which require several processing steps and long manufacturing times, thus making the overall process lengthy and with high cost. Moreover, orodispersible edoxaban compositions known from the prior art made by wet-granulation techniques tend to deliver finished drug formulations with variability with respect to the dissolution rate of edoxaban. Another limitation of the known orodispersible edoxaban compositions is their stability, as during wet-granulation steps edoxaban is subjected to humidity conditions (water or other solvents) or to high temperatures required for drying obtained edoxaban granules. Such harsh conditions result in stability problems of the manufactured orodispersible edoxaban compositions.

For example, EP3549585 discloses orally disintegrating tablets comprising edoxaban, an organic acid, 0.1 to 2.0% w/w of a water-soluble polymer relative to the total weight of the disintegrating tablet, and a disintegrant. Disclosed examples therein, having adequate physicochemical properties such as disintegration time and hardness, were all manufactured by compressing two different types of granules obtained after two distinct wet-granulation processes, i.e. in a first step, edoxaban-containing granules (referred to as "drug-containing granules") were obtained by wet-granulating a mixture comprising the active ingredient with other excipients, employing as a granulating agent an aqueous solution containing 0.1 to 2.0% w/w of a water-soluble polymer, whereas a second step consisted of wet-granulating pharmaceutically acceptable excipients, which yielded a second type of granules (referred to as "rapidly disintegrating granules"). The final step consisted of mixing of both edoxaban-containing type granules and rapidly disintegrating granules with further excipients, and compression to obtain orally disintegrated tablets.

EP3815686 discloses edoxaban granular preparations which are manufactured by two separated and independent granulation processes which yield two different types of granules. Specifically, a first type of granules, which are edoxaban-containing granules (referred to as "drug-containing granules") are obtained by wet-granulating a mixture of edoxaban with other excipients. In a subsequent and separated step, a second type of granules (referred to as "drug-free granules") are obtained by wet-granulating a mixture of xylitol or sorbitol with carmellose sodium, employing as a granulating agent either an aqueous solution containing carmellose sodium or water alone.

In Japan, edoxaban (Lixiana^{®}) was approved by the Japanese Pharmaceuticals and Medical Devices Agency (PMDA) on April 2011. Edoxaban is approved in Japan for the same mentioned indications as for Europe and US. Additionally, it is approved in Japan for the prevention of venous thromboembolism (VTE) in patients undergoing any of the following orthopedic surgeries for the lower limbs: total knee replacement, total hip replacement, and hip fracture surgery.

Besides being available in the form of conventional film-coated tablets, edoxaban (Lixiana^{®}) is also marketed as orally disintegrated tablets in 15, 30 and 60 mg strengths (based on edoxaban free base). ODT's are distinguished from conventional sublingual tablets, buccal tablets and lozenges by their fast disintegration time in the oral cavity, which generally takes around 60 seconds or less. In fact, the FDA Center for Drug Evaluation and Research (CDER) defines orodispersible tablets as: "A solid dosage form containing medicinal substances which disintegrates rapidly, usually within a matter of seconds, when placed upon the tongue". Therefore, it is commonly accepted in the art of developing/manufacturing orodispersible tablets that such tablets, when placed in the mouth, should disintegrate in less than one minute - otherwise, if disintegration takes longer, the attractiveness of orodispersible tablets is lost, as the taste buds of the patients' tongue and other receptors of the oral cavity are extensively exposed to a direct contact with the active ingredient. Moreover, for those patients who are travelling or which have limited access to water, it may be discomfortable to take an orodispersible tablet which does not disintegrate rapidly (i.e. in less than one minute). In this regard, it has been observed that 60 mg strength of Lixiana^{®} orally disintegrated tablets, which is commercially available in Japan, takes longer than 60 seconds to disintegrate completely. This may result in a lower acceptability of said orodispersible dosage form, which ultimately may impact adherence to the treatment and worsening of the clinical pattern.

Edoxaban is a Class 4 substance according to the Biopharmaceuticals Classification System (BCS), with low aqueous solubility and low permeability. Edoxaban exhibits high solubility in a strong acidic aqueous solution, but its solubility decreases in a neutral pH aqueous solution. Lixiana^{®} film-coated tablets and orally disintegrating tablets release edoxaban in immediate-release manner. In particular, it has been determined that both commercially available forms of Lixiana^{®} (conventional tablets and orodispersible tablets) release 85% or more of edoxaban within 15 minutes at pH=1.2.

When developing new orodispersible pharmaceutical dosage forms, it should be borne in mind that it is a standard requirement by regulatory agencies to prove strict bioequivalence criteria of said newly developed orodispersible pharmaceutical dosage form versus the conventional film coated tablet of the reference product. For instance, in Europe, according to the Guideline for Bioequivalence (CPMP/EWP/QWP/1401/98 Rev.1/Corr., 2010), if it cannot be demonstrated that the active ingredient is not absorbed in the oral cavity, bioequivalence for new developed orodispersible tablets must be evaluated in human studies. Therefore, said requirement of bioequivalence poses considerable challenges for the development of new orodispersible tablets.

Therefore, in view of the prior art, there is the need to provide for edoxaban orodispersible pharmaceutical dosage forms having overall improved characteristics. In this regard, there is room for improvement in the prior art for providing edoxaban orodispersible compositions obtainable by a fast, straightforward and low-cost process while having at the same time improved disintegration time, stability, friability and hardness. Furthermore, there is the need to provide for edoxaban orodispersible pharmaceutical dosage forms which, from a regulatory standpoint, exhibit reliable and adequate dissolution profile of edoxaban for resulting in being bioequivalent to Lixiana^{®} immediate-release film-coated tablets.

### Summary of the invention

The inventors of the present invention have been capable of designing new orodispersible pharmaceutical dosage forms comprising edoxaban (or a pharmaceutically acceptable salt thereof or a hydrate of said edoxaban or a hydrate of said edoxaban pharmaceutically acceptable salt), a binder in an amount of from 3.5% to 15.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban (or a pharmaceutically acceptable salt thereof or a hydrate of said edoxaban or a hydrate of said edoxaban pharmaceutically acceptable salt), the binder, the disintegrant, and the one or more further pharmaceutically acceptable excipients.

By manufacturing orodispersible pharmaceutical dosage form of the invention by a direct compression process, the manufacture of a complete batch of edoxaban orodispersible tablets is made within few hours, thus allowing complete production in a single-day shift at the manufacturing site. This represents an improvement with respect to wet-granulation manufacturing processes known in the art, which require more than the standard 8-hours shift in a manufacturing site. In other words, the time employed in the manufacturing of a complete batch of edoxaban orodispersible tablets according to the invention (i.e. by a direct compression process) is less than half as the one required for manufacturing of a complete batch of edoxaban orodispersible tablets according to EP3549585 A1. Thus, direct compression process of the invention provides an improvement in time of manufacturing, leading to a reduction of in economic and energetic manufacturing costs.

Even more, the inventors have surprisingly found that orodispersible pharmaceutical dosage forms of the invention, obtained by direct compression, comprising edoxaban (or a pharmaceutically acceptable salt thereof or a hydrate of said edoxaban or a hydrate of said edoxaban pharmaceutically acceptable salt), a binder in an amount of from 3.5% to 15.0% w/w relative to the total weight of the dosage form, a disintegrant, and one or more further pharmaceutically acceptable excipients, also have overall improved technical characteristics as summarised hereinafter.

Despite the teaching away comments from EP3549585, whereby it is disclosed that if the content of a water-soluble polymer (i.e. a type of binder) is too high - meaning above 3.0% w/w relative to the total weight of the edoxaban orodispersible dosage form - suspending of the final tablet takes too much time and therefore it is not suitable as an orally disintegrating tablet, the inventors have unexpectedly found that orodispersible pharmaceutical dosage forms of the invention, which comprise a binder in high amount (i.e. from 3.5% to 15.0% w/w relative to the total weight of the dosage form), indeed satisfactorily disintegrate rapidly, in particular in less than two minutes, particularly in less than one minute and a half, more particularly in less than one minute, and even more particularly in less than 55 seconds. In particular, the dosage forms of the invention disintegrate in the time range of from two minutes and 5 seconds, preferably in the time range of from one minute and 40 seconds and 10 seconds, more preferably in the time range of from one minute and 30 seconds and 15 seconds, and even more preferably in the time range of from one minute and 15 seconds and 20 seconds.

This disintegration time represents an improvement with respect to 60 mg strength commercially available Lixiana^{®} orally disintegrated tablets, which take longer than one minute and a half to completely disintegrate and which are manufactured by compressing two different types of granules ("edoxaban-containing granules" and "rapidly disintegrating granules") obtained after two distinct and separated wet-granulation processes. Such an improved disintegration time demonstrates that edoxaban orodispersible dosage forms of the invention are suitable dosage forms for replacing conventional Lixiana^{®} film-coated tablets in the treatment of: (i) prevention of stroke and systemic embolism in adult patients with nonvalvular atrial fibrillation (NVAF) with one or more risk factors, and/or (ii) treatment of deep vein thrombosis (DVT) and pulmonary embolism (PE), and prevention of recurrent DVT and PE in adults; and/or (iii) prevention of venous thromboembolism (VTE) in patients undergoing any of the following orthopedic surgeries for the lower limbs: total knee replacement, total hip replacement, and hip fracture surgery.

Edoxaban orodispersible pharmaceutical dosage forms of the invention have adequate hardness and low friability, which are important advantages in order to withstand physical shocks along manufacturing process and for storage and handling transportation. Moreover, edoxaban orodispersible pharmaceutical dosage forms of the invention have an improved stability with respect to commercially available Lixiana^{®} orally disintegrated tablets and Lixiana^{®} film-coated tablets. By manufacturing the orodispersible forms of the invention via direct compression process within the presence of a binder in an amount of from 3.5% to 15.0% w/w relative to the total weight of the dosage form, a disintegrant, and one or more further pharmaceutically acceptable excipients, formation of degradation products can surprisingly be prevented over time. Particularly, edoxaban orodispersible compositions of the invention comply with the strict criteria of impurities limit specifications required by the European or Japanese regulatory agencies.

Furthermore, edoxaban orodispersible pharmaceutical dosage forms of the invention allow to provide reliable and adequate dissolution profile of edoxaban, thus releasing edoxaban in a sufficiently equivalent manner for being bioequivalent to immediate-release film-coated tablets of Lixiana^{®}. In this regard, edoxaban orodispersible compositions of the invention rapidly release at least 85% or more of edoxaban within 15 minutes at pH=1.2. By having such dissolution profile, and thus being sufficiently equivalent to currently marketed Lixiana^{®} conventional tablets, edoxaban orodispersible dosage forms of the invention avoids the risk of being either suprabioequivalent or infrabioequivalent, which may affect the toxicity and/or the efficacy of said orodispersible dosage form. Therefore, orodispersible pharmaceutical dosage forms of the invention constitute a valuable therapeutic tool in the field of anticoagulant medicinal products.

Thus, a first aspect of the present invention relates to an orodispersible pharmaceutical dosage form comprising edoxaban or a pharmaceutically acceptable salt thereof or a hydrate of said edoxaban or a hydrate of said edoxaban pharmaceutically acceptable salt, a binder in an amount of from 3.5% to 15.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

A second aspect of the invention relates to a direct compression process for the preparation of the orodispersible dosage form of the first aspect of the invention, which comprises the steps of: (i) mixing edoxaban or a pharmaceutically acceptable salt thereof or a hydrate of said edoxaban or a hydrate of said edoxaban pharmaceutically acceptable salt with the binder, the disintegrant, and the one or more further pharmaceutically acceptable excipients; (ii) blending the mixture of step (i); (iii) adding one or more lubricants to the mixture of step (ii); (iv) blending the mixture of step (iii); (v) compressing the dry powdered mixture of step (iv) to form an orodispersible pharmaceutical dosage form.

A third aspect of the invention relates to the orodispersible dosage form of the fist aspect of the invention for use in: a) prevention of stroke and systemic embolism in adult patients with nonvalvular atrial fibrillation (NVAF) with one or more risk factors, such as congestive heart failure, hypertension, age ≥ 75 years, diabetes mellitus, prior stroke or transient ischaemic attack (TIA); and/or b) treatment of deep vein thrombosis (DVT) and pulmonary embolism (PE), and for the prevention of recurrent DVT and PE in adults; and/or c) prevention of venous thromboembolism (VTE) in patients undergoing any of the following orthopedic surgeries for the lower limbs: total knee replacement, total hip replacement, and hip fracture surgery.

### Brief description of the drawings

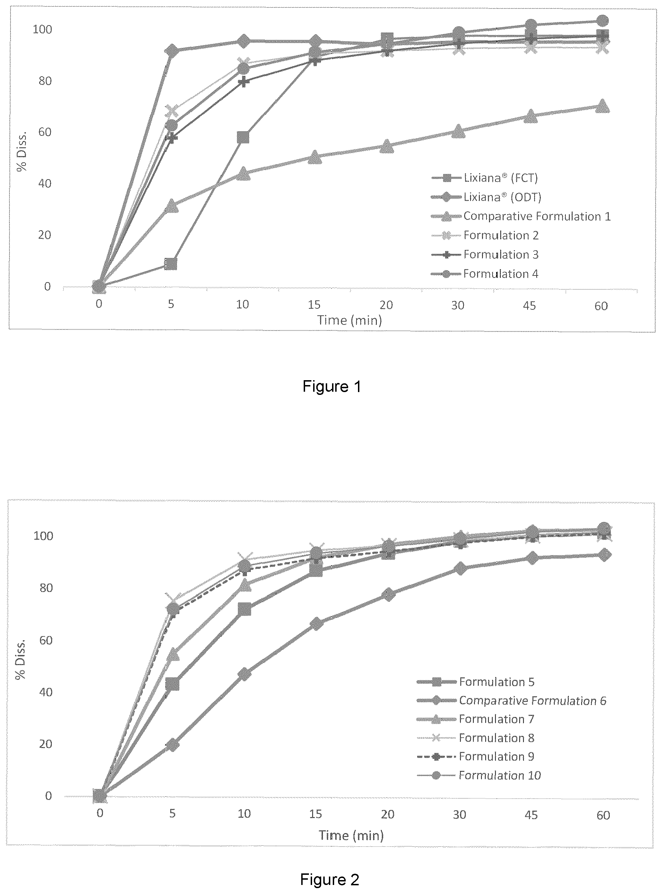

Figure 1 shows the dissolution profiles of: (i) film-coated tablet of Lixiana^{®} (60 mg strength), (ii) a Japanese commercially available orally disintegrating tablet Lixiana^{®} (60 mg strength), (iii) Comparative Formulation 1, (iv) Formulations 2, 3 and 4. Said dissolution profiles were measured according to the following dissolution method: (i) USP Apparatus II (paddle); (ii) speed = 50 rpm; (iii) pH=1.2; (iv) volume = 900 mL; (v) temperature = 37 °C; (vi) N=6. Percentage of dissolution (%D) is expressed in % w/w; time (t) is expressed in minutes (min).
Figure 2 shows the dissolution profiles of: (i) Comparative Formulation 6, (ii) Formulations 5, 7, 8, 9 and 10. Said dissolution profiles were measured according to the following dissolution method: (i) USP Apparatus II (paddle); (ii) speed = 50 rpm; (iii) pH=1.2; (iv) volume = 900 mL; (v) temperature = 37 °C; (vi) N=6. Percentage of dissolution (%D) is expressed in % w/w; time (t) is expressed in minutes (min).

### Detailed description of the invention

The term "active ingredient" or "API" as used herein refers to a pharmaceutically active molecule (e.g. edoxaban) as well as its pharmaceutically acceptable and therapeutically active salts, hydrates, esters, amides, prodrugs, metabolites, enantiomers, polymorphs, analogs, etc. that induce a desired pharmacological or physiological effect. Terms like "active", "active agent", "active pharmaceutical ingredient", "active substance", "drug substance", "active drug" may be used synonymously for "active ingredient".

The term "edoxaban" as used herein corresponds to the International Non-proprietary Name (INN) for (N'-(5-chloropyridin-2-yl)-N-[(1S,2R,4S)-4-(dimethylcarbamoyl)-2-[(5-methyl-6,7-dihydro-4H-[1,3]thiazolo[5,4-c]pyridine-2-carbonyl)amino]cyclohexyl]oxamide) but also as their pharmaceutically acceptable salts and/or hydrates. Pharmaceutically acceptable salts of edoxaban and their hydrates comprise any of a broad range of inorganic and organic acids. Examples of such salts include acid addition salts with a mineral acid such as hydrochloric acid, hydrobromic acid, hydroiodic acid, sulfuric acid, nitric acid, or phosphoric acid, and those with an organic acid such as p-toluenesulfonic acid, methanesulfonic acid, benzenesulfonic acid, benzoic acid, citric acid, acetic acid, propionic acid, oxalic acid, malonic acid, succinic acid, glutaric acid, adipic acid, tartaric acid, maleic acid, malic acid, fumaric acid, mandelic acid. The preferred salts and their hydrates for the purpose of the invention are edoxaban p-toluenesulfonate (also referred to as edoxaban tosylate), edoxaban p-toluenesulfonate monohydrate (also referred to as edoxaban tosylate monohydrate), edoxaban methanesulfonate and edoxaban benzenesulfonate. Among them, the most preferred salt for the purpose of the present invention is edoxaban p-toluenesulfonate monohydrate (also referred to as edoxaban tosylate monohydrate).

The term "orodispersible pharmaceutical dosage form" as used herein encompasses different pharmaceutical forms, such as orodispersible tablets, orodispersible mini-tablets, orodispersible powders, amongst others. The preferred orodispersible pharmaceutical dosage form for the purpose of the present invention is an orodispersible tablet.

The term "orodispersible tablet" as used herein is defined in accordance with the European Pharmacopeia, edition 10.0, page 939, as an uncoated tablet intended to be placed in the mouth where it disperses rapidly before being swallowed, more precisely orodispersible tablets disintegrate within three minutes in the disintegration test. In accordance with this definition the term orodispersible tablet is intended to be a synonym of solid oral dosage forms named as orodispersable tablet, orodisperse tablet, orally disintegrating tablet, orally disintegrated tablet, fast disintegrating tablet, fast dissolving tablet, mouth-dissolving tablets, amongst others.

The term "disintegration test" as used herein is defined in accordance with Test A of the European Pharmacopeia (edition 10.0, page 323), water having pH=7, at 37°C and 30 cycles per minute. The term "complete disintegration" as used herein is defined in accordance with the European Pharmacopeia, edition 10.0, page 323. Disintegration, as defined herein, does not imply complete dissolution of the dosage form or even of its active pharmaceutical ingredient.

The term "disintegrate" as used herein is defined as the action whereby a solid dosage form is brought from a solid state to a state of complete disintegration.

The term "dissolution profile" as used herein refers to dissolution over time of edoxaban from the dosage form of the invention. Hereinafter, the dissolution profile is measured in weight of dissolved edoxaban (as free base) per initial weight of edoxaban in the dosage form (calculated based on the weight of edoxaban free base), and it is expressed in weight percentage (% w/w). Unless otherwise stated, hereinafter, the dissolution profiles have been measured under two different pH conditions: a) at pH=1.2; and b) at pH=6.8. Dissolution profile measured at pH=1.2 are determined using a USP Apparatus II (paddle), placing the dosage form in 900 mL (for edoxaban strengths of 60 mg and 30 mg) and in 500 mL (for edoxaban strength of 15 mg), at 37 °C, stirring at 50 revolutions per minute and N=6. Dissolution profile measured at pH=6.8 are determined using a USP Apparatus II (paddle), placing the dosage form in 900 mL (for edoxaban strengths of 60 mg and 30 mg) and in 500 mL (for edoxaban strength of 15 mg), at 37 °C, stirring at 50 revolutions per minute and N=6.

The term "edoxaban immediately release" or "edoxaban released in immediate-release manner" as used herein refer to dissolution of at least 85% of edoxaban as free base, expressed in weight percentage (% w/w) per initial weight of edoxaban in the dosage form (calculated based on the weight of edoxaban free base), within 15 minutes at pH=1.2, measured as described in the above paragraph.

The term "friability" as used herein refers to the tendency for a tablet to chip, crumble or break during handling. The test of friability is carried out following the guidelines of the European Pharmacopeia, edition 10.0, pp. 336-337. A maximum loss of mass not greater than 1.0 % is considered acceptable for most products.

The term "pharmaceutically acceptable excipient" (also named as "excipients") refers to a substance formulated alongside with the active pharmaceutical ingredient of a medicinal product and includes all kind of pharmaceutically acceptable compounds commonly used in pharmaceutical compositions and in particular orodispersible tablets. The term "pharmaceutically acceptable excipients" comprise diluents, binders, disintegrants, lubricants, organic acids, sweeteners, glidants, colorants and flavor agents and mixtures thereof.

The term "diluent" as used herein is defined as a pharmaceutical acceptable excipient that is used as diluent in pharmaceutical compositions. The term "diluent" comprises one or combinations of two or more selected from the group of mannitol, maltol, sorbitol, maltitol, xylitol, isomalt, erythritol, lactose, starch and its derivatives such as pregelatinized starch, cellulose and its derivatives, in particular microcrystalline cellulose, and calcium phosphate.

Starch and pregelatinized starch are generally considered as diluents but they may have also some disintegrant-like properties and/or some binder-like properties. Therefore, both could be considered as multi-functional excipients. However, in the context of the present invention, starch and pregelatinized starch are defined exclusively as diluents. In other words, in the context of the present invention, the term "binder" and the term "disintegrant" do not encompass starch nor pregelatinized starch.

The term "binder" as used herein is defined as a pharmaceutical acceptable excipient that hold the ingredients together in pharmaceutical compositions. Binders are the agents used to increase the cohesion of the powdery particles or granules during the compression, in order to obtain pharmaceutical forms with a defined hardness. Binders ensure that tablets and granules can be formed with required mechanical strength.

However, in the context of the present invention, binders are defined exclusively as agents used to increase the cohesion of the powdery particles. In other words, taking into account that orodispersible pharmaceutical dosage forms of the invention are only obtainable by a direct compression process (no granulation process is involved at all), the term "binder" does not encompass agents used to prepare binder solutions for manufacturing granules.

Additionally, in the context of the present invention, the term "binder" shall not be understood as a "coating agent", as a coating agent serves the purpose of coating the pharmaceutical dosage form (e.g. tablets) whereas binders as used herein in the present invention are pharmaceutical acceptable excipients that hold powdery particles together in pharmaceutical compositions. In other words, the term "binder" in the context of the present invention do not encompass agents which are used as coating agents or in a coating process.

Binders, in the context of the present invention, are classified into 2 classes: a) "natural binders" and b) "cellulosic polymers".

The term "natural binders" as used herein is defined as a pharmaceutical acceptable excipient which is a natural polymer binder or salt thereof, preferably selected from the group of alginic acid, sodium alginate, gelatin, Guar gum, Arabic gum, Candelilla wax, Carnauba wax and mixtures thereof

The term "cellulosic" or "cellulosic polymer" as used herein is defined as substance made from cellulose, relating to cellulose or being a chemical derivative of cellulose. Examples of "cellulosic polymers" are hydroxypropyl methyl cellulose, hydroxypropyl cellulose, hydroxyethyl methyl cellulose, hydroxyethyl cellulose, methyl cellulose, ethyl cellulose, sodium carboxymethyl cellulose and mixtures thereof. The preferred cellulosic polymers for the purpose of the present invention are selected from the group of consisting of: hydroxypropyl methyl cellulose, hydroxypropyl cellulose, hydroxyethyl methyl cellulose, hydroxyethyl cellulose, methyl cellulose, ethyl cellulose, sodium carboxymethyl cellulose. The most preferred are hydroxypropyl methyl cellulose and hydroxypropyl cellulose.

The term "disintegrant" as used herein is defined as a pharmaceutical acceptable excipient that is used as disintegrant in pharmaceutical compositions. The term "disintegrant" comprises one or combinations of two or more selected from the group of: crospovidone, croscarmellose sodium, carmellose calcium, carmellose, calcium silicate and sodium starch glycolate and mixtures thereof.

The term "lubricant" as used herein is defined as a pharmaceutical acceptable excipient that is used as lubricant in pharmaceutical compositions. The term "lubricant" comprises one or combinations of two or more selected from the group of talc, sodium benzoate, sodium stearyl fumarate, calcium stearate, magnesium stearate, zinc stearate, glyceryl behenate, stearic acid and glyceryl monostearate; more particularly, such lubricant is selected from the group of sodium stearyl fumarate and magnesium stearate.

The term "organic acid" as used herein is defined as a pharmaceutical acceptable excipient that is an organic compound that is acidic and can be used in pharmaceutical compositions. Acidic means that the pH in an aqueous solution is less than 7. Organic acids include carboxylic acids, sulfonic acids and enols, or salts thereof. The term "organic acid" comprises one or combinations of two or more selected from the group of adipic acid, aspartic acid, ascorbic acid, alginic acid, benzoic acid, citric acid, anhydrous citric acid, glutamic acid, succinic acid, tartaric acid, sorbic acid, lactic acid, fumaric acid, maleic acid, malonic acid, malic acid, oxalic acid, galactaric acid, gluconic acid, and glucuronic acid; more particularly, such organic acid is selected from the group of citric acid, anhydrous citric acid, tartaric acid and fumaric acid.

The term "sweetener" as used herein is defined as a pharmaceutical acceptable excipient that is used as sweetener in pharmaceutical compositions. The term "sweetener" comprises one or combinations of two or more selected from the group of aspartame, potassium acesulfame (Acesulfame K), sodium saccharinate, neohesperidine dihydrochalcone, sucralose, sucrose, fructose, monoammonium glycyrrhizinate and mixtures thereof.

The term "glidant" as used herein is defined as a pharmaceutical acceptable excipient that is used as glidant in pharmaceutical compositions. The term "glidant" comprises one or combinations of two or more selected from the group of colloidal silicon dioxide, hydrophobic colloidal silica, magnesium oxide, magnesium silicate, magnesium trisilicate and mixtures thereof.

The term "colorant" as used herein is defined as a pharmaceutical acceptable excipient that is used as colorant in pharmaceutical compositions. The term "colorant" comprises one or combinations of two or more selected from the group of Food Yellow No.5, Food Red No. 2, and Food Blue No.2; food lake dyes, Yellow Ferric Oxide, iron sesquioxide, titanium oxide, β-carotene, riboflavin and mixtures thereof; more particularly, such colorant is selected from the group of Yellow Ferric Oxide, iron sesquioxide and titanium oxide.

The term "flavouring agent" as used herein is defined as a pharmaceutical acceptable excipient that is used as flavouring agent in pharmaceutical compositions. The term "flavouring agent" comprises one or combinations of two or more selected from the group of cherry, raspberry, apricot, pear, strawberry, bitter masker, pineapple, lemon, honey, mint garden, orange, peppermint, menthol, black currant, banana, red fruits, wild berries and caramel flavour; more particularly, such flavouring agent is selected from the group of cherry, apricot, mint and honey flavour.

As it is mentioned above, an aspect of the present invention relates to an orodispersible pharmaceutical dosage form comprising edoxaban or a pharmaceutically acceptable salt thereof or a hydrate of said edoxaban or a hydrate of said edoxaban pharmaceutically acceptable salt, a binder in an amount of from 3.5% to 15.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

According to the invention, the amount of the binder present in the orodispersible pharmaceutical dosage form may vary between 3.5% to 15.0% w/w relative to the total weight of the dosage form, preferably between 3.5% to 12.0% w/w relative to the total weight of the dosage form, more preferably between 4.5% to 10.0% w/w relative to the total weight of the dosage form, and even more preferably between 4.5% to 8.0% or 4.5% to 6.0% w/w relative to the total weight of the dosage form. The orodispersible pharmaceutical dosage form of the invention may comprise the binder in 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0% w/w relative to the total weight of the dosage form.

According to the invention, the amount of one or more disintegrants present in the orodispersible pharmaceutical dosage form may range between 7.5% to 17.5% w/w relative to the total weight of the dosage form, or between 7.5% to 12.5% w/w relative to the total weight of the dosage form, or between 12.5% to 17.5% w/w relative to the total weight of the dosage form. The orodispersible pharmaceutical dosage form of the invention may comprise one or more disintegrants in 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5% w/w relative to the total weight of the dosage form.

According to the invention, the orodispersible pharmaceutical dosage form may comprise an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form.

More preferably, the orodispersible pharmaceutical dosage form of the invention may comprise an organic acid at 0.5%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0% w/w relative to the total weight of the dosage form.

The organic acid used in the orodispersible pharmaceutical dosage form according to the invention is not particularly limited, but examples thereof include adipic acid, aspartic acid, ascorbic acid, alginic acid, benzoic acid, citric acid, anhydrous citric acid, glutamic acid, succinic acid, tartaric acid, sorbic acid, lactic acid, fumaric acid, maleic acid, malonic acid, malic acid, oxalic acid, galactaric acid, gluconic acid, and glucuronic acid; more preferably examples thereof include citric acid, anhydrous citric acid, tartaric acid and fumaric acid.

In an embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 15.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 12.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 4.5% to 10.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 15.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 12.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 4.5% to 10.0% w/w relative to the total weight of the dosage form, a disintegrant, and one or more further pharmaceutically acceptable excipients, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.21 to 1:1.35, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.21 to 1:1.35, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.21 to 1:1.35, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.24 to 1:1.05, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.24 to 1:1.05, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.24 to 1:1.05, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.31 to 1:0.7, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.31 to 1:0.7, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a binder in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.31 to 1:0.7, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.21 to 1:1.35, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.21 to 1:1.35, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.21 to 1:1.35, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.24 to 1:1.05, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.24 to 1:1.05, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.24 to 1:1.05, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.31 to 1:0.7, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.31 to 1:0.7, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a binder in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the binder is of from 1:0.31 to 1:0.7, wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 15.0% w/w relative to the total weight of the dosage form, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 12.0% w/w relative to the total weight of the dosage form, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 4.5% to 10.0% w/w relative to the total weight of the dosage form, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 15.0% w/w relative to the total weight of the dosage form, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 12.0% w/w relative to the total weight of the dosage form, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In a more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 4.5% to 10.0% w/w relative to the total weight of the dosage form, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 15.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 12.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In an even more particular embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 4.5% to 10.0% w/w relative to the total weight of the dosage form, a disintegrant, and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 15.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form, and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 12.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 4.5% to 10.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 15.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 12.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 4.5% to 10.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 15.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 3.5% to 12.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt, a cellulosic polymer in an amount of from 4.5% to 10.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 15.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 12.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 4.5% to 10.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.21 to 1:1.35, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 15.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 12.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 4.5% to 10.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.24 to 1:1.05, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 15.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 3.5% to 12.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, the orodispersible pharmaceutical dosage form of the invention comprises edoxaban or a pharmaceutically acceptable salt thereof or a hydrate of said edoxaban or a hydrate of said edoxaban pharmaceutically acceptable salt in amount per dosage form of from 15 to 60 mg, based on the weight of edoxaban free base, a cellulosic polymer in an amount of from 4.5% to 10.0% w/w relative to the total weight of the dosage form, a disintegrant, an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form and one or more further pharmaceutically acceptable excipients, wherein the weight-to-weight ratio of edoxaban, calculated based on the weight of edoxaban free base, to the cellulosic polymer is of from 1:0.31 to 1:0.7, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the cellulosic polymer, the disintegrant, the organic acid and the one or more further pharmaceutically acceptable excipients.

In a preferred embodiment, edoxaban or a pharmaceutically acceptable salt thereof or a hydrate of said edoxaban or a hydrate of said edoxaban pharmaceutically acceptable salt is edoxaban comprised in the orodispersible pharmaceutical dosage form of the invention is edoxaban p-toluenesulfonate monohydrate.

In a preferred embodiment, the orodispersible pharmaceutical dosage form of the invention is an orodispersible tablet.

In a more preferred embodiment, the orodispersible pharmaceutical dosage form of the invention is an an orodispersible tablet comprising edoxaban p-toluenesulfonate monohydrate, a binder in an amount of from 3.5% to 15.0% w/w relative to the total weight of the orodispersible tablet, a disintegrant, and one or more further pharmaceutically acceptable excipients, wherein said orodispersible tablet is obtainable by a direct compression process of a dry powdered mixture comprising edoxaban p-toluenesulfonate monohydrate, the binder, the disintegrant, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a binder in an amount of from 3.5% to 12.0% w/w relative to the total weight of the orodispersible tablet, a disintegrant, and one or more further pharmaceutically acceptable excipients, wherein said orodispersible tablet is obtainable by a direct compression process of a dry powdered mixture comprising edoxaban p-toluenesulfonate monohydrate, the binder, the disintegrant, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a binder in an amount of from 4.5% to 10.0% w/w relative to the total weight of the orodispersible tablet, a disintegrant, and one or more further pharmaceutically acceptable excipients, wherein said orodispersible tablet is obtainable by a direct compression process of a dry powdered mixture comprising edoxaban p-toluenesulfonate monohydrate, the binder, the disintegrant, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a cellulosic polymer in an amount of from 3.5% to 15.0% w/w relative to the total weight of the orodispersible tablet, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible tablet is obtainable by a direct compression process of a dry powdered mixture comprising edoxaban p-toluenesulfonate monohydrate, the cellulosic polymer, the disintegrant, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a cellulosic polymer in an amount of from 3.5% to 12.0% w/w relative to the total weight of the orodispersible tablet, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible tablet is obtainable by a direct compression process of a dry powdered mixture comprising edoxaban p-toluenesulfonate monohydrate, the cellulosic polymer, the disintegrant, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a cellulosic polymer in an amount of from 4.5% to 10.0% w/w relative to the total weight of the orodispersible tablet, a disintegrant, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, and wherein said orodispersible tablet is obtainable by a direct compression process of a dry powdered mixture comprising edoxaban p-toluenesulfonate monohydrate, the cellulosic polymer, the disintegrant, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a cellulosic polymer in an amount of from 3.5% to 15.0% w/w relative to the total weight of the orodispersible tablet, a disintegrant, an organic acid in an amount of 4% w/w relative to the total weight of the orodispersible tablet, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, wherein the organic acid is citric acid or anhydrous citric acid or tartaric acid, and wherein said orodispersible tablet is obtainable by a direct compression process of a dry powdered mixture comprising edoxaban p-toluenesulfonate monohydrate, the cellulosic polymer, the disintegrant, the organic acid, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a cellulosic polymer in an amount of from 3.5% to 12.0% w/w relative to the total weight of the orodispersible tablet, a disintegrant, an organic acid in an amount of 4% w/w relative to the total weight of the orodispersible tablet, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, wherein the organic acid is citric acid or anhydrous citric acid or tartaric acid, and wherein said orodispersible tablet is obtainable by a direct compression process of a dry powdered mixture comprising edoxaban p-toluenesulfonate monohydrate, the cellulosic polymer, the disintegrant, the organic acid, and the one or more further pharmaceutically acceptable excipients.

In a more preferred embodiment, the orodispersible pharmaceutical dosage form of the invention is an an orodispersible tablet comprising edoxaban p-toluenesulfonate monohydrate, a cellulosic polymer in an amount of from 4.5% to 10.0% w/w relative to the total weight of the orodispersible tablet, a disintegrant, an organic acid in an amount of 4% w/w relative to the total weight of the orodispersible tablet, and one or more further pharmaceutically acceptable excipients, wherein the cellulosic polymer is selected from the group consisting of hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, wherein the organic acid is citric acid or anhydrous citric acid or tartaric acid, and wherein said orodispersible tablet is obtainable by a direct compression process of a dry powdered mixture comprising edoxaban p-toluenesulfonate monohydrate, the cellulosic polymer, the disintegrant, the organic acid, and the one or more further pharmaceutically acceptable excipients.

As it is mentioned above, a second aspect of the invention relates to a direct compression process for the preparation of the orodispersible dosage form of the first aspect of the invention, which comprises the steps of: (i) mixing edoxaban or a pharmaceutically acceptable salt thereof or a hydrate of said edoxaban or a hydrate of said edoxaban pharmaceutically acceptable salt with the binder, the disintegrant, and the one or more further pharmaceutically acceptable excipients; (ii) blending the mixture of step (i); (iii) adding one or more lubricants to the mixture of step (ii); (iv) blending the mixture of step (iii); (v) compressing the dry powdered mixture of step (iv) to form an orodispersible pharmaceutical dosage form.

In an embodiment, the process for the preparation of the orodispersible pharmaceutical dosage form of the invention comprises the steps of: (i) mixing edoxaban or a pharmaceutically acceptable salt thereof or a hydrate of said edoxaban or a hydrate of said edoxaban pharmaceutically acceptable salt with a binder, a disintegrant, and one or more further pharmaceutically acceptable excipients; (ii) blending the mixture of step (i); (iii) adding one or more lubricants to the mixture of step (ii); (iv) blending the mixture of step (iii); (v) compressing the dry powdered mixture of step (iv) to form an orodispersible pharmaceutical dosage form.

In an embodiment, the process for the preparation of the orodispersible pharmaceutical dosage form of the invention provides an orodispersible tablet, wherein the process comprises the steps of: (i) mixing edoxaban or a pharmaceutically acceptable salt thereof or a hydrate of said edoxaban or a hydrate of said edoxaban pharmaceutically acceptable salt with a binder, a disintegrant, and one or more further pharmaceutically acceptable excipients; (ii) blending the mixture of step (i); (iii) adding one or more lubricants to the mixture of step (ii); (iv) blending the mixture of step (iii); (v) compressing the dry powdered mixture of step (iv) to form an orodispersible tablet.

In a particular embodiment, the process for the preparation of the orodispersible pharmaceutical dosage form of the invention provides an orodispersible tablet, wherein the process comprises the steps of: (i) mixing edoxaban p-toluenesulfonate monohydrate with a binder, a disintegrant, and one or more further pharmaceutically acceptable excipients; (ii) blending the mixture of step (i); (iii) adding one or more lubricants to the mixture of step (ii); (iv) blending the mixture of step (iii); (v) compressing the dry powdered mixture of step (iv) to form an orodispersible tablet.

All the embodiments disclosed above for the orodispersible pharmaceutical dosage form of the invention apply also for the preparation process.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim and shall not be construed as limiting the scope of the claim. Furthermore, the present invention covers all possible combinations of particular and preferred embodiments described herein.

### Examples

### Physicochemical characterization of commercially available Lixiana^{®} orally disintegrated tablets in Japan

A sample of 60 mg strength of Lixiana^{®} orally disintegrated tablets commercially available in Japan was purchased and further characterized:
- Description: Uncoated tablet - oval shape with dividing line
- Strength: 60 mg (based on edoxaban free base)
- Average weight: 362.76 mg
- Diameter: 13.406 mm
- Thickness: 4.78 mm
- Hardness: 96.3 N
- Disintegration time: 1 min 45 sec (i.e. 105 seconds)

Disintegration time was obtained by following Test A of the European Pharmacopeia, (edition 10.0, page 323), with water having pH=7, at 37°C and 30 cycles per minute.

Thus, as it can be observed, disintegration time of currently on the market Lixiana^{®} orally disintegrated tablets is far away from the gold-standard in the field - i.e. disintegration time in around 60 seconds or less. In fact, the observed disintegration time almost double the generally accepted maximum value of around 60 seconds.

### Example 1: General manufacturing process of orodispersible tablets of the invention

Edoxaban (or a pharmaceutically acceptable salt thereof or a hydrate of said edoxaban or a hydrate of said edoxaban pharmaceutically acceptable salt), a binder and one or more disintegrants are weighed and sieved through a 1 mm mesh and further added to a suitable bin blender (Servo-lift bowl). The following further excipients may be optionally added to the mixture present in the bin blender: a diluent, an organic acid, a sweetener, a glidant, a colorant and a flavouring agent. The mixture is blended between 10 and 25 minutes at 34 rpm. Then, a lubricant is weighed and sieved through a 1 mm mesh and further added to the blended mixture. The obtained mixture is blended 5 minutes at 34 rpm. The obtained dry powdered mixture is compressed in a rotatory press into the desired orodispersible tablets of the invention. A general composition is provided below in Table 1.

**Table 1: general composition of the orodispersible tablets of the invention**

| ***Component*** | | **% *w*/*w*** |
|---|---|---|
| Edoxaban or edoxaban salt or edoxaban salt hydrate | | 3.5 - 25 % |
| Binder | | 3.5 - 15% |
| Disintegrant | | 7.5 - 17.5 % |
| Further excipients: | | |
| | • Diluent | 25 - 50 % |
| | • Organic acid | 0.1 - 20 % |
| | • Sweetener | 0.50 - 1 % |
| | • Glidant | 0.25 - 0.75 % |
| | • Colorant | 0.01 - 0.50 % |
| | • Flavour agent | 3 - 7 % |
| | • Lubricant | 0.50 - 2.50 % |

### Example 2: Orodispersible tablets compositions according to the invention

Formulations 2 to 5 - as representative examples according to the invention - are shown in Tables 2-3. Said Formulations 2 to 5 were similarly prepared according to the manufacturing process described in Example 1. Orodispersible tablets of Formulations 2 to 5 comprised edoxaban p-toluenesulfonate monohydrate (i.e. edoxaban tosylate monohydrate) as active pharmaceutical ingredient. Comparative Formulation 1 (which comprises 2% w/w binder) and Comparative Formulation 6 (which comprises 20% w/w binder) are also shown below in Tables 2-3.

**Table 2: Composition of Comparative Formulation 1, and Formulations 2 and 3.**

| **Component** | **Comparative Formulation 1** | | **Formulation 2** | | **Formulation 3** | |
|---|---|---|---|---|---|---|
| | % w/w | mg | % w/w | mg | % w/w | mg |
| Edoxaban tosylate monohydrate* | 19.24 | 80.80 | 19.24 | 80.80 | 22.44 | 80.80 |
| D-mannitol | 34.44 | 144.66 | 30.73 | 129.06 | 26.71 | 96.14 |
| Starch | 15.00 | 63.00 | 15.00 | 63.00 | 17.50 | 63.00 |
| Crospovidone | 10.00 | 42.00 | 10.00 | 42.00 | 9.98 | 35.92 |
| HPMC | 2.00 | 8.40 | - | - | 6.67 | 24.00 |
| HPC | - | - | 5.71 | 24.00 | - | - |
| Sodium croscarmellose | 7.50 | 31.50 | 7.50 | 31.50 | 8.75 | 31.50 |
| Aspartame | 0.73 | 3.08 | 0.73 | 3.08 | 0.86 | 3.08 |
| Citric acid | 4.00 | 16.80 | 4.00 | 16.80 | 4.67 | 16.80 |
| Colloidal silicone dioxide | 0.50 | 2.10 | 0.50 | 2.10 | 0.58 | 2.10 |
| Magnesium stearate | 1.50 | 6.30 | 1.50 | 6.30 | 1.75 | 6.30 |
| Yellow ferric oxide | 0.09 | 0.36 | 0.09 | 0.36 | 0.10 | 0.36 |
| Honey flavour | 5.00 | 21.00 | 5.00 | 21.00 | - | - |
| Total | 100.00 | 420.00 | 100.00 | 420.00 | 100.00 | 360.00 |

| | | | | | | |
|---|---|---|---|---|---|---|
| **80.80 mg of edoxaban tosylate monohydrate correspond to 60.00 mg of edoxaban free base.* | | | | | | |

**Table 3: Composition of Formulations 4 and 5 and Comparative Formulation 6.**

| **Component** | **Formulation 4** | | **Formulation 5** | | **Comparative Formulation 6** | |
|---|---|---|---|---|---|---|
| | % w/w | mg | % w/w | mg | % w/w | mg |
| Edoxaban tosylate monohydrate* | 19.24 | 80.80 | 19.24 | 80.80 | 19.24 | 80.80 |
| D-mannitol | 35.73 | 150.06 | 32.89 | 138.14 | 16.44 | 69.06 |
| Starch | 15.00 | 63.00 | 15.00 | 63.00 | 15.00 | 63.00 |
| Crospovidone | 5.00 | 21.00 | 8.55 | 35.92 | 10.00 | 42.00 |
| HPMC | 5.71 | 24.00 | 10.00 | 42.00 | 20.00 | 84.00 |
| Sodium croscarmellose | 7.50 | 31.50 | 7.50 | 31.50 | 7.50 | 31.50 |
| Aspartame | 0.73 | 3.08 | 0.73 | 3.08 | 0.73 | 3.08 |
| Citric acid | 4.00 | 16.80 | 4.00 | 16.80 | 4.00 | 16.80 |
| Colloidal silicone dioxide | 0.50 | 2.10 | 0.50 | 2.10 | 0.50 | 2.10 |
| Magnesium stearate | 1.50 | 6.30 | 1.50 | 6.30 | 1.50 | 6.30 |
| Yellow ferric oxide | 0.09 | 0.36 | 0.09 | 0.36 | 0.09 | 0.36 |
| Mint flavour | 5.00 | 21.00 | - | - | | |
| Honey flavour | - | - | - | - | 5.00 | 21.00 |
| Total | 100.00 | 420.00 | 100.00 | 420.00 | 100.00 | 420.00 |

| | | | | | | |
|---|---|---|---|---|---|---|
| **80.80 mg of edoxaban tosylate monohydrate correspond to 60.00 mg of edoxaban free base.* | | | | | | |

### Example 3. Characterization of orodispersible tablets according to the invention: Disintegration test, hardness, thickness, friability test

Formulations 2 to 5 (according to the invention) and Comparative Formulations 1 and 6, as described in Tables 2-3, were tested for disintegration time following Test A of the European Pharmacopeia (edition 10.0, page 323), water having pH=7, at 37°C and 30 cycles per minute. The results are shown in Table 4.

Formulations 2 to 5 (according to the invention) and Comparative Formulations 1 and 6 were also submitted for friability test in accordance with the guidelines of the European Pharmacopeia, edition 10.0, pp. 336-337. The results are shown in Table 4. Formulations 2 to 5 (according to the invention) and Comparative Formulations 1 and 6 were also characterized by their hardness and thickness.

**Table 4: Characterization of orodispersible tablets according to the invention**

| **Orodispersible tablet** | **Disintegration time (sec.)** | **Friability (%)** | **Hardness (N)** | **Thickness (mm)** |
|---|---|---|---|---|
| Comparative Formulation 1 | 38 | 0.09 | 103 | 4.97 |
| Formulation 2 | 43 | 0.05 | 121 | 4.98 |
| Formulation 3 | 44 | 0.09 | 78 | 4.52 |
| Formulation 4 | 61 | 0.05 | 103 | 4.91 |
| Formulation 5 | 43 | 0.08 | 121 | 5.01 |
| Comparative Formulation 6 | 110 | 0.06 | 114 | 5.02 |

As shown in Table 4, all orodispersible tablets of Formulations 2 to 5 (according to the invention) complied with the requirement of being orodispersible tablets, in accordance with the definition given by the European Pharmacopeia, edition 10.0, page 939. Furthermore, all disintegrated in around 60 seconds or less - thus being considered as fast/quickly orally disintegrating tablets. Comparative Formulation 6, although it can formally be defined as orodispersible tablet according to the definition given by the European Pharmacopeia, it needed almost two minutes to completely disintegrate.

Moreover, all orodispersible tablets of Formulations 2 to 5 (according to the invention) complied with the requirement of having a friability of less than 1 % in accordance with guidelines of the European Pharmacopeia.

### Example 4. Dissolution profile of orodispersible tablets according to the invention

Dissolution profile of Formulations 2 to 5 (according to the invention), Comparative Formulations 1 and 6, a commercially available film-coated tablet of Lixiana^{®} (60 mg strength) and a Japanese commercially available orally disintegrating tablet Lixiana^{®} (60 mg strength), were measured according to the following dissolution method: (i) USP Apparatus II (paddle); (ii) speed = 50 rpm; (iii) pH=1.2; (iv) volume = 900 mL; (v) temperature = 37 °C; (vi) N=6. The results are shown in Tables 5-6.

**Table 5: Dissolution profiles of Lixiana^{®} film-coated tablet (60 mg strength), Lixiana^{®} orally disintegrating tablet (60 mg strength), Comparative Formulation 1 and Formulation 2.**

| | **Lixiana^{®} (FCT)** | | **Lixiana^{®} (ODT)** | | **Comparative Formulation 1** | | **Formulation 2** | |
|---|---|---|---|---|---|---|---|---|
| T (min) | Dis. (%) | SD (%) | Dis. (%) | SD (%) | Dis. (%) | SD (%) | Dis. (%) | SD (%) |
| 0 | 0.0 | 0.0 | 0,0 | 0,0 | 0.0 | 0.0 | 0.0 | 0.0 |
| 5 | 8.8 | 3.8 | 91,9 | 6,7 | 31.7 | 1.2 | 68.7 | 3.1 |
| 10 | 58.3 | 11.9 | 95,9 | 0,7 | 44.4 | 2.2 | 87.2 | 2.2 |
| **15** | **89.9** | **6.6** | **95,9** | **0,8** | **51.0** | **2.9** | **91.2** | **1.4** |
| 20 | 97.0 | 1.2 | 95,0 | 0,8 | 55.4 | 3.3 | 92.4 | 1.2 |
| 30 | 98.3 | 0.6 | 96,0 | 0,7 | 61.3 | 3.5 | 93.5 | 1.0 |
| 45 | 98.5 | 0.6 | 96,1 | 0,7 | 67.3 | 3.3 | 94.0 | 1.0 |
| 60 | 98.7 | 0.6 | 96,3 | 0,8 | 71.4 | 3.3 | 94.1 | 1.1 |

**Table 6: Dissolution profiles of Formulations 3, 4, 5 and Comparative Formulation 6.**

| | **Formulation 3** | | **Formulation 4** | | **Formulation 5** | | **Comparative Formulation 6** | |
|---|---|---|---|---|---|---|---|---|
| T (min) | Dis. (%) | SD (%) | Dis. (%) | SD (%) | Dis. (%) | SD (%) | Dis. (%) | SD (%) |
| 0 | 0.0 | 0.0 | 0.0 | 0.0 | 0.0 | 0.0 | 0.0 | 0.0 |
| 5 | 58.1 | 12.6 | 62.9 | 8.3 | 43.2 | 4.69 | 19.9 | 3.5 |
| 10 | 80.2 | 5.3 | 85.1 | 5.9 | 72.2 | 11.1 | 47.2 | 6.8 |
| **15** | **88.6** | **3.6** | **91.7** | **3.5** | **87.1** | **2.0** | **66.8** | **8.1** |
| 20 | 92.4 | 2.5 | 95.1 | 2.7 | 93.9 | 0.9 | 78.1 | 8.5 |
| 30 | 95.4 | 2.3 | 99.6 | 2.1 | 98.6 | 0.2 | 88.2 | 6.2 |
| 45 | 97.4 | 2.1 | 102.7 | 2.1 | 101.0 | 0.3 | 92.5 | 4.5 |
| 60 | 98.6 | 1.9 | 104.4 | 2.0 | 102.1 | 0.2 | 93.8 | 3.6 |

The dissolution profiles show that orodispersible pharmaceutical dosage forms of the invention (Formulations 2 to 5) rapidly release at least 85% or more of edoxaban within 15 minutes at pH=1.2. When binder is present in low amount (i.e. 2% w/w in Comparative Formulation 1) or too high amount (20% w/w in Comparative Formulation 6), edoxaban is not immediate-released (i.e. less than 85% dissolved within 15 minutes at pH=1.2).

By having such dissolution profile, and thus being sufficiently equivalent to currently marketed Lixiana^{®} conventional tablets, edoxaban orodispersible dosage forms of the invention (Formulations 2 to 5) avoids the risk of being either suprabioequivalent or infrabioequivalent, which may affect the toxicity and/or the efficacy of said orodispersible dosage form.

### Example 5: Further orodispersible tablets compositions according to the invention

Further orodispersible tablets of Tables 7-8 were similarly prepared according to the manufacturing process described in Example 1.

**Table 7: Composition of formulations 7 and 8.**

| **Component** | **Formulation 7** | | **Formulation 8** | |
|---|---|---|---|---|
| | % w/w | mg | % w/w | mg |
| Edoxaban tosylate monohydrate* | 19.24 | 80.80 | 19.24 | 80.80 |
| D-mannitol | 35.63 | 149.66 | 33.72 | 141.64 |
| Pregelatinized starch | 15.00 | 63.00 | - | - |
| Starch | - | - | 15.00 | 63.00 |
| Crospovidone | 5.10 | 21.40 | 10.00 | 42.00 |
| HPMC | 5.71 | 24.00 | 5.71 | 24.00 |
| Sodium croscarmellose | 7.50 | 31.50 | 7.50 | 31.52 |
| Aspartame | 0.73 | 3.08 | 0.73 | 3.08 |
| Tartaric acid | 4.00 | 16.80 | - | - |
| Citric acid | - | - | 1.00 | 4.20 |
| Colloidal silicone dioxide | 0.50 | 2.10 | 0.50 | 2.10 |
| Magnesium stearate | 1.50 | 6.30 | 1.50 | 6.30 |
| Yellow ferric oxide | 0.09 | 0.36 | 0.09 | 0.36 |
| Mint flavour | - | - | 5.00 | 21.00 |
| Honey flavour | 5.00 | 21.00 | - | - |
| Total | 100.00 | 420.00 | 100.00 | 420.00 |

| | | | | |
|---|---|---|---|---|
| **80.80 mg of edoxaban tosylate monohydrate correspond to 60.00 mg of edoxaban free base.* | | | | |

**Table 8: Composition of formulations 9 and 10.**

| **Component** | **Formulation 9** | | **Formulation 10** | |
|---|---|---|---|---|
| | % w/w | mg | % w/w | mg |
| Edoxaban tosylate monohydrate* | 19.24 | 80.80 | 19.24 | 80.80 |
| D-mannitol | 40.73 | 171.08 | 30.73 | 129.06 |
| Starch | 15.00 | 63.00 | 15.00 | 63.00 |
| Crospovidone | 5.00 | 21.00 | 10.00 | 42.00 |
| HPMC | 5.71 | 24.00 | 5.71 | 24.00 |
| Sodium croscarmellose | 2.50 | 10.48 | 7.50 | 31.50 |
| Aspartame | 0.73 | 3.08 | 0.73 | 3.08 |
| Citric acid | 4.00 | 16.80 | 4.00 | 16.80 |
| Colloidal silicone dioxide | 0.50 | 2.10 | 0.50 | 2.10 |
| Magnesium stearate | 1.50 | 6.30 | 1.50 | 6.30 |
| Yellow ferric oxide | 0.09 | 0.36 | 0.09 | 0.36 |
| Mint flavour | 5.00 | 21.00 | 5.00 | 21.00 |
| Total | 100.00 | 420.00 | 100.00 | 420.00 |

| | | | | |
|---|---|---|---|---|
| **80.80 mg of edoxaban tosylate monohydrate correspond to 60.00 mg of edoxaban free base.* | | | | |

### Example 6. Characterization of further orodispersible tablets according to the invention: Disintegration test, hardness, thickness, friability test

Formulations 7 to 10 (according to the invention), as described in Tables 7-8, were tested for disintegration time following Test A of the European Pharmacopeia (edition 10.0, page 323), water having pH=7, at 37°C and 30 cycles per minute. The results are shown in Table 9.

Formulations 7 to 10 were also submitted for friability test in accordance with the guidelines of the European Pharmacopeia, edition 10.0, pp. 336-337. The results are shown in Table 9. Formulations 7 to 10 were also characterized by their hardness and thickness.

**Table 9: Characterization of further orodispersible tablets according to the invention**

| **Orodispersible tablet** | **Disintegration time (sec.)** | **Friability (%)** | **Hardness (N)** | **Thickness (mm)** |
|---|---|---|---|---|
| Formulation 7 | 56 | 0.23 | 97 | 4.90 |
| Formulation 8 | 42 | 0.08 | 97 | 4.98 |
| Formulation 9 | 62 | 0.11 | 104 | 4.88 |
| Formulation 10 | 52 | 0.09 | 100 | 5.03 |

As shown in Table 9, all orodispersible tablets of Formulations 7 to 10 (according to the invention) complied with the requirement of being orodispersible tablets, in accordance with the definition given by the European Pharmacopeia, edition 10.0, page 939. Furthermore, all disintegrate in around 60 seconds or less - thus being considered as fast/quickly orally disintegrating tablets.

Moreover, all orodispersible tablets of Formulations 7 to 10 (according to the invention) complied with the requirement of having a friability of less than 1 % in accordance with guidelines of the European Pharmacopeia.

### Example 7. Dissolution profile of further orodispersible tablets according to the invention

The dissolution profile of Formulations 7 to 10 (according to the invention) was measured according to the following dissolution method: (i) USP Apparatus II (paddle); (ii) speed = 50 rpm; (iii) pH=1.2; (iv) volume = 900 mL; (v) temperature = 37 °C; (vi) N=6. The results are shown in Table 10.

**Table 10: Dissolution profiles of Formulations 7, 8, 9 and 10.**

| | **Formulation 7** | | **Formulation 8** | | **Formulation 9** | | **Formulation 10** | |
|---|---|---|---|---|---|---|---|---|
| T (min) | Dis. (%) | SD (%) | Dis. (%) | SD (%) | Dis. (%) | SD (%) | Dis. (%) | SD (%) |
| 0 | 0.0 | 0.0 | 0.0 | 0.0 | 0.0 | 0.0 | 0.0 | 0.0 |
| 5 | 54.9 | 13.7 | 75.4 | 8.0 | 71.0 | 5.8 | 72.0 | 6.8 |
| 10 | 81.6 | 8.6 | 91.2 | 3.5 | 87.3 | 5.1 | 88.8 | 3.1 |
| **15** | **92.2** | **4.2** | **95.0** | **2.7** | **92.1** | **4.5** | **93.8** | **2.6** |
| 20 | 97.3 | 1.1 | 96.9 | 2.5 | 94.8 | 3.8 | 96.6 | 3.2 |
| 30 | 100.6 | 1.2 | 99.3 | 2.4 | 98.1 | 3.0 | 99.6 | 3.6 |
| 45 | 102.8 | 1.9 | 100.9 | 2.5 | 100.7 | 2.6 | 102.4 | 3.7 |
| 60 | 103.5 | 2.4 | 101.8 | 2.5 | 102.0 | 2.7 | 103.8 | 3.2 |

The dissolution profiles show that orodispersible pharmaceutical dosage forms of the invention (Formulations 7 to 10) rapidly release at least 85% or more of edoxaban within 15 minutes at pH=1.2.

By having such dissolution profile, and thus being sufficiently equivalent to currently marketed Lixiana^{®} conventional tablets, edoxaban orodispersible dosage forms of the invention (Formulations 7 to 10) avoids the risk of being either suprabioequivalent or infrabioequivalent, which may affect the toxicity and/or the efficacy of said orodispersible dosage form.

### Example 8: Forced degradation studies of formulations of the invention

As representative Formulations according to the invention, Formulations 4 and 10 were subjected to forced degradation studies under different temperature and Relative Humidity (RH) conditions. For comparative purposes, samples of Lixiana^{®} orally disintegrated tablets (ODT) commercially available in Japan and of Lixiana^{®} film-coated tablets (FCT) were also analysed. The forced degradation studies were performed under the following conditions: (a) accelerated conditions at 40 °C/75% RH; (b) thermal degradation at 50 °C. The following impurities were quantified (See Table 11): (i) the amount of 2-(((1S,2R,4S)-4-(dimethylcarbamoyl)-2-(5-methyl-4,5,6,7-tetrahydrothiazolo[5,4-c]pyridine-2-carboxamido) cyclohexyl)amino)-2-oxoacetic acid (also referred to as "oxoacetic acid impurity"), which is the main degradation impurity of edoxaban by hydrolysis; (ii) the amount of the major unknown impurity; (iii) the amount of total impurities. All values are expressed as % w/w.

**Table 11: Forced degradation studies.**

| **Dregadat. conditions** | **Impurities** | **Formulation 4** | **Formulation 10** | **Lixiana^{®} ODT** | **Lixiana^{®} FCT** |
|---|---|---|---|---|---|
| T0 | Oxoacetic imp. | 0.01 % | 0.02 % | 0.04 % | 0.03 % |
| | Major unknown imp. | 0.03 % | 0.04 % | 0.04 % | 0.03 % |
| | Total imp. | 0.09 % | 0.11 % | 0.17 % | 0.19 % |
| 40 °C/75% RH | Oxoacetic imp. | 0.03 % | 0.02 % | 0.03 % | 0.02 % |
| | Major unknown imp. | 0.03 % | 0.03 % | 0.04 % | 0.08 % |
| | Total imp. | 0.11 % | 0.10 % | 0.20 % | 0.25 % |
| 50 °C | Oxoacetic imp. | 0.02 % | 0.02 % | 0.03 % | 0.02 % |
| | Major unknown imp. | 0.03 % | 0.04 % | 0.04 % | 0.08 % |
| | Total imp. | 0.10 % | 0.12 % | 0.20 % | 0.25 % |

As shown in Table 11, representative Formulations according to the invention, prepared by a direct compression process (Formulations 4 and 10) are more stable under forced degradation conditions than commercially available Lixiana^{®} orally disintegrated tablets and Lixiana^{®} film-coated tablets. In particular, the total impurities present in Formulations 4 and 10 are half the amount present in the commercially available products, which are manufactured by wet-granulation.

### Example 9: Stability of formulations of the invention

Formulation 11 has an identical quantitative composition than Formulation 10 (see Table 8), except that mint flavour (5% w/w, 21 mg) has been replaced by apricot flavour (5% w/w, 21 mg). Formulation 11 has been identically prepared than Formulation 10.

Formulation 11 was subjected to stability studies, at conditions of 25 °C and 60% Relative Humidity (RH), and it was analyzed at 1, 2 and 3 months. For comparative purposes, samples of Lixiana^{®} film-coated tablets (FCT) were also analysed under the same conditions. The following impurities were quantified (See Table 12): (i) the amount of 2-(((1S,2R,4S)-4-(dimethylcarbamoyl)-2-(5-methyl-4,5,6,7-tetrahydrothiazolo[5,4-c]pyridine-2-carboxamido) cyclohexyl)amino)-2-oxoacetic acid (also referred to as "oxoacetic acid impurity"), which is the main degradation impurity of edoxaban by hydrolysis; (ii) the amount of the major unknown impurity; (iii) the amount of total impurities. All values are expressed as % w/w.

**Table 12: Stability studies under 25 °C and 60% RH.**

| **Time** | **Impurities** | **Formulation 11** | **Lixiana^{®} FCT** |
|---|---|---|---|
| 1 month | Oxoacetic imp. | 0.01 % | 0.01 % |
| | Major unknown imp. | 0.02 % | 0.03 % |
| | Total imp. | 0.08 % | 0.06 % |
| 2 months | Oxoacetic imp. | 0.01 % | ND |
| | Major unknown imp. | 0.04 % | 0.06 % |
| | Total imp. | 0.12 % | 0.14 % |
| 3 months | Oxoacetic imp. | ND | ND |
| | Major unknown imp. | 0.02 % | 0.04 % |
| | Total imp. | 0.07 % | 0.12 % |

| | | | |
|---|---|---|---|
| *ND stands for non-detected* | | | |

As shown in Table 12, Formulation 11 which was prepared by a direct compression process is more stable, under stability studies at 25 °C and 60% RH, than commercially available Lixiana^{®} film-coated tablet. In particular, at 2 and 3-month time analysis, major unknown impurity and total number of impurities was significantly lower in Formulation 11 according to the present invention than commercially available product, which is manufactured by wet-granulation.

### Example 10: Manufacturing time comparison between orodispersible formulations manufactured according to the invention and orodispersible formulations of the prior art

As a representative Formulation according to the invention, the manufacturing time needed for obtaining Formulation 10 was compared to the manufacturing time needed for obtaining formulation disclosed in [example 1-2] of EP3549585 A1 (See Table 13).

Formulation 10, having a quantitative formulation as described in Example 5 (Table 8), was manufactured according to process described in Example 1 - all the following components were separately weighed and sieved through a 1 mm mesh and further added to a bin blender (Servo-lift bowl): 404.00 g of edoxaban tosylate monohydrate, 645.30 g of D-mannitol, 315.00 g of starch, 210.00 g of crospovidone, 120.00 g of hydroxypropyl methyl cellulose (HPMC), 157.50 g of sodium croscarmellose, 15.40 g of aspartame, 84.00 g of citric acid, 10.50 of colloidal silicone dioxide, 1.80 g of yellow ferric oxide and 150.00 g of mint flavour. Weighing, sieving and adding all components took 60 minutes. The resulting mixture was blended for 15 minutes. Then, 31.50 g of magnesium stearate were weighed and sieved through a 1 mm mesh and further added to the blended mixture. This took 5 minutes. The obtained mixture, containing the magnesium stearate was blended for 5 minutes. The obtained dry powdered mixture was compressed in a rotatory press into the desired orodispersible tablets of the invention. Compression into tablets took 180 minutes.

Formulation disclosed in [example 1-2] of EP3549585 A1 was prepared according to the described process therein (i.e. obtaining two different types of granules after two distinct wet-granulation processes). Weighing and adding all components to the granulator dryer equipment took 60 minutes. Granulation for obtaining the drug-containing granules (i.e. edoxaban-containing granules) took 60 minutes, plus another 60 minutes for drying. Then, weighing and adding all components to the equipment for the second granulation took 60 minutes. Granulation for obtaining the rapidly disintegrating granules took 60 minutes, plus another 60 minutes for drying. The drug-containing granules and rapidly disintegrating granules were mixed and blended for 15 minutes. Then, magnesium stearate was weighed and sieved through a 1 mm mesh and further added to the blended mixture. This took 5 minutes. The obtained mixture, containing the magnesium stearate was blended for 5 minutes. The obtained dry powdered mixture was compressed in a rotatory press into orodispersible tablets, requiring 180 minutes.

**Table 13. Manufacturing time comparison between orodispersible formulations manufactured according to the invention and orodispersible formulations of the prior art.**

| **Manufacturing step** | **Time needed according to the process of the invention** | **Time needed according to process disclosed in** EP3549585 A1 |
|---|---|---|
| Weighing, sieving, and adding of components | 60 min | 60 min |
| Granulation for obtaining drug-containing granules | - | 60 min |
| Drying of drug-containing granules | - | 60 min |
| Weighing, sieving, and adding of components for a second granulation | - | 60 min |
| *Granulation for obtaining rapidly disintegrating granules* | - | *60 min* |
| Drying of rapidly disintegrating granules | - | 60 min |
| Blend | 15 min | 15 min |
| Weighing, sieving, and adding of magnesium stearate | 5 min | 5 min |
| Blend of the mixture containing magnesium stearate | 5 min | 5 min |
| Compression into orodispersible tablets | 180 min | 180 min |
| Total time | **265 min** | **565 min** |
| Relative time | **47%** | **100 %** |

As shown in Table 13, the time employed in the manufacturing of a complete batch of edoxaban orodispersible tablets according to the invention (i.e. by a direct compression process) is less than half as the one required for manufacturing of a complete batch of edoxaban orodispersible tablets according to EP3549585 A1. Thus, direct compression process of the invention provides an improvement in time of manufacturing, leading to a reduction of in economic and energetic manufacturing costs.

### Citation List

EP3549585
EP3815686
European Pharmacopeia, edition 10.0, page 939
European Pharmacopeia, edition 10.0, page 323
European Pharmacopeia, edition 10.0, pp. 336-337
Guideline for Bioequivalence (CPMP/EWP/QWP/1401/98 Rev.1/Corr., 2010)

## Claims

1. An orodispersible pharmaceutical dosage form comprising edoxaban or a pharmaceutically acceptable salt thereof or a hydrate of said edoxaban or a hydrate of said edoxaban pharmaceutically acceptable salt, a binder in an amount of from 3.5% to 15.0% w/w relative to the total weight of the dosage form, a disintegrant, and one or more further pharmaceutically acceptable excipients,
wherein said orodispersible pharmaceutical dosage form is obtainable by a direct compression process of a dry powdered mixture comprising edoxaban or a pharmaceutically acceptable salt thereof or a hydrate of said edoxaban or a hydrate of said edoxaban pharmaceutically acceptable salt, the binder, the disintegrant, and the one or more further pharmaceutically acceptable excipients.

2. The orodispersible pharmaceutical dosage form according to claim 1, wherein said orodispersible pharmaceutical dosage form is disintegrated in less than 3 minutes, particularly in less than 2 minutes, and more particularly in less than 1 minute, wherein the disintegration test was performed using a European Pharmacopeia disintegration apparatus A, placing the dosage form in water having pH=7 at 37 °C and 30 cycles per minute.

3. The orodispersible pharmaceutical dosage form according to any of the claims 1-2, wherein the binder is present in an amount of from 3.5% to 12.0% w/w relative to the total weight of the dosage form, preferably in an amount of 4.5% to 10.0% w/w relative to the total weight of the dosage form.

4. The orodispersible pharmaceutical dosage form according to any of the claims 1-3, wherein the amount of edoxaban or a pharmaceutically acceptable salt thereof or a hydrate of said edoxaban or a hydrate of said edoxaban pharmaceutically acceptable salt per dosage form is of from 15 to 60 mg, based on the weight of edoxaban free base.

5. The orodispersible pharmaceutical dosage form according to any of the claims 1-4, wherein the weight-to-weight ratio of edoxaban, calculated based on the weight of edoxaban free base, to the binder is of from 1:0.21 to 1:1.35, preferably of from 1:0.24 to 1:1.05, and more preferably of from 1:0.31 to 1:0.7.

6. The orodispersible pharmaceutical dosage form according to any of the claims 1-5, wherein the binder is a cellulosic polymer.

7. The orodispersible pharmaceutical dosage form according to claim 6, wherein the cellulosic polymer is selected from the group consisting of: hydroxypropyl methyl cellulose, hydroxypropyl cellulose, hydroxyethyl methyl cellulose, hydroxyethyl cellulose, methyl cellulose, ethyl cellulose, sodium carboxymethyl cellulose, preferably being selected from hydroxypropyl methyl cellulose and hydroxypropyl cellulose.

8. The orodispersible pharmaceutical dosage form according to any of the claims 1-7, wherein said dosage form comprises one or more disintegrants in an amount of from 7.5% to 17.5% w/w relative to the total weight of the dosage form.

9. The orodispersible pharmaceutical dosage form according to any of the claims 1-8, wherein the one or more disintegrants is/are selected from the group consisting of: crospovidone, croscarmellose sodium, carmellose calcium, carmellose, calcium silicate and sodium starch glycolate and mixtures thereof.

10. The orodispersible pharmaceutical dosage form according to any of the claims 1-9, wherein said dosage form further comprises an organic acid in an amount of from 0.1% to 20% w/w relative to the total weight of the dosage form, preferably in in an amount of from 0.5% to 15% w/w relative to the total weight of the dosage form, more preferably in in an amount of from 0.75% to 10% w/w relative to the total weight of the dosage form, and even more preferably in an amount of from 1% to 8% w/w relative to the total weight of the dosage form.

11. The orodispersible pharmaceutical dosage form according to claim 10, wherein said dosage form comprises an organic acid selected from the group consisting of: adipic acid, aspartic acid, ascorbic acid, alginic acid, benzoic acid, citric acid, anhydrous citric acid, glutamic acid, succinic acid, tartaric acid, sorbic acid, lactic acid, fumaric acid, maleic acid, malonic acid, malic acid, oxalic acid, galactaric acid, gluconic acid, and glucuronic acid, preferably being selected from citric acid, anhydrous citric acid, tartaric acid and fumaric acid.

12. The orodispersible pharmaceutical dosage form according to claim 10, wherein the organic acid is citric acid or anhydrous citric acid or tartaric acid and wherein said organic acid is present in an amount of 4% w/w relative to the total weight of the dosage form.

13. The orodispersible pharmaceutical dosage form according to any of the claims 1-11, wherein the percentage-to-percentage ratio of organic acid to one or more disintegrants is of from 1:1.875 to 1:17.5.

14. The orodispersible pharmaceutical dosage form according to any of the claims 1-13, wherein the direct compression process comprises the steps of:
(i) mixing edoxaban or a pharmaceutically acceptable salt thereof or a hydrate of said edoxaban or a hydrate of said edoxaban pharmaceutically acceptable salt with the binder, the disintegrant, and the one or more further pharmaceutically acceptable excipients,
(ii) blending the mixture of step (i),
(iii) adding one or more lubricants to the mixture of step (ii),
(iv) blending the mixture of step (iii),
(v) compressing the dry powdered mixture of step (iv) to form an orodispersible pharmaceutical dosage form.

15. The orodispersible pharmaceutical dosage form according to any of claims 1-14 for use in the treatment of: a) prevention of stroke and systemic embolism in adult patients with nonvalvular atrial fibrillation (NVAF) with one or more risk factors, such as congestive heart failure, hypertension, age ≥ 75 years, diabetes mellitus, prior stroke or transient ischaemic attack (TIA); and/or b) treatment of deep vein thrombosis (DVT) and pulmonary embolism (PE), and for the prevention of recurrent DVT and PE in adults; and/or c) prevention of venous thromboembolism (VTE) in patients undergoing any of the following orthopedic surgeries for the lower limbs: total knee replacement, total hip replacement, and hip fracture surgery.

16. A process for the preparation of the orodispersible pharmaceutical dosage form as defined according to any of claims 1-14, which comprises the steps of:
(i) mixing edoxaban or a pharmaceutically acceptable salt thereof or a hydrate of said edoxaban or a hydrate of said edoxaban pharmaceutically acceptable salt with the binder, the disintegrant, and the one or more further pharmaceutically acceptable excipients,
(ii) blending the mixture of step (i),
(iii) adding one or more lubricants to the mixture of step (ii),
(iv) blending the mixture of step (iii),
(v) compressing the dry powdered mixture of step (iv) to form an orodispersible pharmaceutical dosage form.
